(19) Europäisches Patentamt
European Patent Office
Office européen des brevets

(11) Veröffentlichungsnummer: **0 007 515**
**B1**

# (12) EUROPÄISCHE PATENTSCHRIFT

(45) Veröffentlichungstag der Patentschrift: **10.03.82**

(21) Anmeldenummer: **79102370.8**

(22) Anmeldetag: **11.07.79**

(51) Int. Cl.³: **C 07 J 1/00**, C 07 J 41/00, C 07 J 31/00 //C07C69/716, C07C121/75, C07C143/68

(54) **7α-Methyl-Östrogene und Verfahren zu deren Herstellung sowie deren Verwendung zur Weiterverarbeitung.**

(30) Priorität: **21.07.78 DE 2832606**

(43) Veröffentlichungstag der Anmeldung: **06.02.80 Patentblatt 80/3**

(45) Bekanntmachung des Hinweises auf die Patenterteilung: **10.03.82 Patentblatt 82/10**

(84) Benannte Vertragsstaaten: **AT BE CH DE FR GB IT LU NL SE**

(56) Entgegenhaltungen:
**FR - A - 2 169 856**
**FR - A - 2 204 624**
**US - A - 4 064 173**

(73) Patentinhaber: **SCHERING AKTIENGESELLSCHAFT Berlin und Bergkamen**
**Müllerstrasse 170/178 Postfach 65 0311**
**D-1000 Berlin 65 (DE)**

(72) Erfinder: **Sauer, Gerhard, Dr.**
**Königsbacher Zeile 41 A**
**D-1000 Berlin 28 (DE)**
Erfinder: **Eder, Ulrich, Dr.**
**Zeltinger Strasse 17**
**D-1000 Berlin 28 (DE)**
Erfinder: **Haffer, Gregor, Dr.**
**Alt Mariendorf 36**
**D-1000 Berlin 42 (DE)**
Erfinder: **Neef, Günter, Dr.**
**Darmstädter Strasse 9**
**D-1000 Berlin 15 (DE)**
Erfinder: **Wiechert, Rudolf, Prof. Dr.**
**Petzower Strasse 8A**
**D-1000 Berlin 39 (DE)**

EP 0 007 515 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# 7$\alpha$-Methyl-Östrogene und Verfahren zu zu deren Herstellung sowie deren Verwendung zur Weiterverarbeitung

Die Erfindung betrifft die in den Ansprüchen gekennzeichneten Gegenstände.

Die 7$\alpha$-Methyl-1,3,5(10),9-östratriene der allgemeinen Formel I sind neue Verbindungen, die als Zwischenprodukte zur Herstellung von pharmakolgisch wirksamen Steroiden verwendet werden können.

Unter niederen Alkylgruppen $R_1$ und $R_2$ sollen Alkylgruppen mit 1—4 C-Atomen verstanden werden, wie beispielsweise Methyl, Äthyl, Propyl, Isopropyl, Butyl, wobei die Methyl- und Äthylgruppe bevorzugt ist. Bedeutet $R_3$ einen neideren oder mittleren Alkylrest, so kommen insbesondere Alkylreste mit 4—8 C-Atomen in Betracht, wobei die Hexylgruppe bevorzugt ist. Verwendbar sind jedoch auch niedere Alkylgruppen mit 1—4 C-Atomen.

Liegt $R_3$ in der Bedeutung eines substituierten Arylrestes vor, so ist der Phenylrest als bevorzugt anzusehen. Der Phenylrest kann zusätzlich substituiert sein, vorzugsweise durch eine Methylgruppe.

Als funktionell geschützte Hydroxymethylgruppen X kommen Ester- und Ätherreste in Betracht, die insbesondere im alkalischem oder schwach saurem Reaktionsmedium beständig sind. Vorzugsweise sind Alkoxymethylengruppen mit 1 bis 4 Kohlenstoffatomen im Alkoxyrest geeignet, beispielsweise seien genannt: der Methoxy- Äthoxy- Propyloxy-, Butyloxy- oder Isopropyloxyrest, wobei der tert.-Butyloxyrest bevorzugt ist. Darüber hinaus geeignet sind auch gemischte Acetale, wie zum Beispiel Tetrahydropyranyläther oder Äthoxyäthyläther.

Die neuen Verbindungen der allgemeinen Formel I eignen sich insbesondere zur Herstellung von 7$\alpha$-Methyl-östratrienen, die als Zwischenprodukte für pharmakologisch wirksame Steroide bekannt sind. (Dt-Patent 1 593 509).

Die Reduktion der Carbonylgruppe erfolgt nach an sich bekannten Methoden, beispielsweise durch eine Reduktion nach Wolff-Kishner oder Clemmensen.

Die Reaktionen werden in einem polaren Lösungsmittel wie zum Beispiel Triäthylenglycol u.a. bei Temperaturen von 100°C bis 230°C durchgeführt.

Die Überführung der Verbindungen der allgemeinen Formel IX in pharmakolgisch wirksame Verbindungen, insbesondere mit östrogener und ovulationshemmender Wirkung, erfolgt nach an sich bekannten Verfahren.

Beispielsweise wird die funktionell geschützte 17$\beta$-Hydroxymethylengruppe X in eine freie Hydroxymethylengruppe überführt und anschließend zur 17-Keto-Verbindung oxidiert. Anschließend kann der 1,3-Diäther — wie bekannt — zu einem 7$\alpha$-Methyl-$\Delta^{1,3,5(10)}$-östratrien-17-on-1,3-diacetat umgewandelt werden, dessen Herstellung und Weiterverarbeitung in der Dt-Patentschrift 1 593 509 beschrieben ist. Werden die funktionellen Schutzgruppen in 17- und 3-Stellung abgespalten, so erhält man 7$\alpha$-Methyl-östradiol, dessen Herstellung in der deutschen Patentschrift 1 443 684 beschrieben ist.

Die Herstellung der neuen Verbindungen der allgemeinen Formel I nach dem erfindungsgemäßen Verfahren und die Weiterreaktion zu den pharmakologische wirksamen Verbindungen erfolgt in so guten Ausbeuten und so wenigen Reaktionsschritten, daß in unerwarteter Weise eine ökonomische Herstellung dieser wichtigen Verbindungen ermöglicht wird.

Führt man den Reaktionsschritt a) des erfindungsgemäßen Verfahrens in wasserfreiem Medium durch, so verwendet man als Deprotonierungsmittel bevorzugt Alkalihydride, Alkalialkoholate, Alkaliamide u.a., wobei Alkalihydride bevorzugt sind. Beispielsweise seien genannt Natriumhydrid, Kaliumhydrid, Kalium-tert.-Butylat, Lithiumhydrid, Lithiumamid, Triphenylmethylkalium usw.. Geeignete Lösungsmittel sind unpolare Lösungsmittel, wie beispielsweise Toluol, Benzol, Xylol u.a. oder polare Lösungsmittel wie Alkoholate (z.B. Methanol, Äthanol, Butanol u.a.) und deren Gemische.

Wird die Reaktion in einem Zweiphasensystem durchgeführt, so kann man unpolare Lösungsmittel wie zum Beispiel Toluol, Benzol, Heptan u.a. und polare Lösungsmittel, beispielsweise Äther (z.B. Diäthyläther, Diisopropyläther u.a.) und chlorierte Kohlenwasserstoffe (z.B. Methylenchlorid, Chloroform, Dichloräthylen, Tetrachloräthan, Chlorbenzol u.a.) verwenden. Dazu gibt man zweckmäßigerweise den üblichen Transferkatalysator und unterschichtet mit einer wäßrigen Base.

Als Transferkatalysator ist beispielsweise Benzyl-triäthyl-ammoniumchlorid, Tetrabutylammonium-hydrogensulfat, Benzyl-trimethyl-ammonium-hydroxyd u.a. geeignet.

Als wäßrige Basen können beispielsweise Alkalihydroxydlösungen, wie zum Beispiel Na-, K-, Lihydroxydlösungen eingesetzt werden. Hierbei kann man Basenlösungen in 10—70 %igen Konzentrationen verwenden, die im Mengenverhältnis 10:1 bis 1:10 zum organischen Lösungsmittel eingesetzt werden können.

Für diesen Reaktionsschritt ist es vorteilhaft, bei Raumtemperatur bis zur Siedetemperatur des Lösungsmittels, vorzugsweise bei +10°C bis +50°C zu arbeiten.

Das erhaltene Isomerisierungsgemisch in 6-Stellung kann ohne Auftrennung in die nächste Reaktionsstufe eingesetzt werden. Die Cyclisierung in der Verfahrensstufe b) wird vorzugsweise in Gegenwart von sauren Katalysatoren wie beispielsweise Mineralsäuren, Sulfonsäure, Lewissäuren oder stark dissoziierende Carbonsäuren, vorgenommen. Beispielsweise seien genannt: Ameisensäure, Monofluoressigsäure, Trichloressigsäure, Oxalsäure, Malonsäure, Salzsäure, Bromwasserstoffsäure,

Schwefelsäure, Benzolsulfonsäure, p-Toluolsulfonsäure oder Bortrifluorid. Besonders bevorzugt sind Mineralsäuren, wie beispielsweise Salzsäure, Schwefelsäure und Phosphorsäure. Geeignete protische Lösungsmittel für die Cyclisierung sind Alkohle wie zum Beispiel Methanol, Äthanol, iso-Propanol, Butanol u.a. oder Ketone, wie zum Beispiel Aceton, Methyläthylketon u.a., oder Carbonsäuren, wie zum Beispiel Ameisensäure, Essigsäure, Propionsäure oder dipolare aprotische Lösungsmittel, wie zum Beispiel Dimethylformamid, Dimethylacetamid, N-Methylpyrrolidon, Hexamethylphosphorsäuretriamid. Die Cyclisierung kann sowohl bei tiefer Temperatur — etwa ab 0°C — als auch bei erhöhter Temperatur — bis etwa 150°C durchgeführt werden. Vorzugsweise erfolgt die Cyclisierung bei einer Reaktionstemperatur von 10 bis 60°C.

Wird als Ausgangsprodukt für die Cyclisierungsreaktion das 6-Isomerengemisch eingesetzt, so kann gewünschtenfalls nach der Cyclisierung eine Trennung der Isomeren nach den üblichen Methoden erfolgen, wie zum Beispiel durch Umkristallisation oder Chromatographie. Vorzugsweise wird jedoch das Lösungsmittel so gewählt, daß das schwerer lösliche $6\alpha$-Isomere kristallin anfällt. Inwieweit der Cyclisierungsablauf durch die Einführung der obengenannten 6-Substituenten beeinflußt wird, ließ sich auch von einem Fachmann nicht vorhersagen. Es ist daher überraschend, daß als Cyclisierungsprodukt in ca. 77 %iger Ausbeute die $6\alpha$-substituierte Verbindung auf einfache Weise aus dem Reaktionsgemisch isoliert werden kann.

In der Verfahrensstufe c) werden die Verbindungen der allgemeinen Formel V in Gegenwart von Edelmetallkatalysatoren hydriert. Als Edelmetallkatalysatoren kommen Pd- und Pt-Katalysatoren, gegebenenfalls auf Trägern, wie beispielsweise $CaCO_3$, $SrCO_3$, $BaSO_4$, Aktivkohle u.a. in Betracht, wobei insbesondere Pd-Kohle bevorzugt ist.

Die Hydrierung kann in protischen Lösungsmitteln wie Alkoholen, zum Beispiel Methanol, Äthanol, n-Propanol, i-Propanol, n-Butanol u.a., in unpolaren Lösungsmitteln wie zum Beispiel Benzol, Toluol u.a., in polaren Lösungsmitteln, wie Äther, Ketonen u.a. durchgeführt werden.

Die Hydrierung kann sowohl bei Raumtemperatur als auch bei erhöhter Temperatur durchgeführt werden; bevorzugt ist eine Reaktionstemperatur von 0°C bis 50°C. Die Hydrierung kann sowohl unter Normaldruck als auch bei erhöhtem Druck vorgenommen werden. Vorzugsweise erfolgt die Hydrierung bei 1—80 Atm.

Vorteilhafterweise wird die nach der Verfahrensstufe b) in sehr guten Ausbeuten kristallin anfallende $6\alpha$-substituierte Verbindung äquilibriert und hydriert.

Die Äquilibrierung kann mit an sich bekannten Mitteln vorgenommen werden, zum Beispiel durch Behandeln mit Basen wie beispielsweise Alkalimetallhydriden wie Li-, Na-, K-Hydrid oder Alkalimetallalkoholaten wie Na-, K-Methylat, K-tert.-Butylat oder Alkalimetallhydroxyden wie NaOH, KOH u.a.. Die Äquilibrierung wird gleichzeitig mit der Hydrierung durchgeführt.

Als Hydrierungsprodukt resultiert ein Gemisch von Stereoisomeren der allgemeinen Formel VI.

Die Oxydation des Isomerengemisches der allgemeinen Formel VI gemäß Verfahrensstufe d), kann nach den dem Fachmann bekannten Verfahren, wie zum Beispiel durch Oxidation mit Luft oder Sauerstoff, der gegebenenfalls mit Stickstoff verdünnt sein kann, vorgenommen werden.

Die Oxydation mit Sauerstoff kann in wasserfreiem Medium in Gegenwart einer starken Base, wie zum Beispiel Alkalihydride, wie Na-, K-, Li-Hydrid oder Alkaliamiden, wie Li-Diisopropylamid, Li-hexamethyl-disilylamid, Li-, Na-, K-Amid wie NaOH, KOH oder Alkalialkoholaten, wie Li-, Na-, K-methylat, -äthylat u.a. durchgeführt werden.

Für die Reaktion geeignete inerte Lösungsmittel sind beispielsweise polare Äther wie Tetrahydrofuran, Dioxan, 1,2-Dimethoxyäthan oder dipolare aprotische Lösungsmittel wie Dimethylformamid, N,N'-Dimethylacetamid, N-Methylpyrrolidon oder unpolare Lösungsmittel wie Benzol, Toluol u.a. und deren Gemische.

Die Reaktion wird bei Temperaturen von —78°C bis 100°C, vorzugsweise bei —20°C bis 50°C, durchgeführt.

Wird die Oxidation als Zweiphasenreaktion ausgeführt, so leitet man Sauerstoff oder Luft in ein Gemisch der folgenden Zusammensetzung:

Als organische Lösungsmittel können unpolare Lösungsmittel wie zum Beispiel Toluol, Benzol, Heptan u.a. und polare Lösungsmittel, wie beispielsweise Äther (z.B. Diäthyläther, Diisopropyläther u.a.) und chlorierte Kohlenwasserstoffe (z.B. Methylenchlorid, Chloroform, Dichloräthylen, Tetrachloroäthan, Chlorbenzol u.a.), als wäßrige Basen können Alkalihydroxydlösungen, wie beispielweise Natrium- Kalium, Lithium-hydroxydlösungen, und zwar in 10—70%igen Konzentrationen, eingesetzt werden.

Die Einführung der 7-Methylgruppe gemäß Verfahrensstufe e) erfolgt nach den dem Fachmann bekannten Methoden. Beispielsweise wird mit Methyljodid in Gegenwart einer starken Base wie zum Beispiel Alkalihydrid, wie Na-, K-, Li-Hydrid oder Alkaliamiden, wie Lithiumdiispropylamid, Licyclohexylisopropylamid oder Li-hexamethyl-di-silylamid in einem inerten Lösungsmittel alkyliert. Als Lösungsmittel kommen beispielsweise in Betracht: unpolare Lösungsmittel, wie Benzol, Toluol, Hexan, Pentan oder Äther, wie Glyme, Tetrahydrofuran, Dioxan, Diäthyläther oder polare aprotische Lösungsmittel, wie Dimethylformamid, Dimethylacetamid, Hexamethylphosphorsäuretriamid u.a., oder Gemische dieser Lösungsmittel.

Die Alkylierung wird üblicherweise bei Temperaturen von —78°C bis 100°C durchgeführt, wobei —50°C bis 20°C bevorzugt sind.

Die isomeren Alkylierungsprodukte können nach den üblichen Methoden aufgetrennt werden, wie zum Beispiel durch Umkristallisation oder Chromatographie.

Das erhaltene Rohprodukt kann jedoch auch ohne Auftrennung in der nächsten Reaktionsstufe eingesetzt werden.

Beispiel 1

a) 17$\beta$ - tert. - Butoxy - 6$\alpha$ - cyan - 1,3 - dimethoxy - 9,10 - seco - 1,3,5 - (10) - östra - trien - 9 - on und 17$\beta$ - tert. - Butoxy - 6$\beta$ - cyan - 1,3 - dimethoxy - 9,10 - seco - 1,3,5(10) - östratrien - 9 - on

Man wäscht eine Kaliumhydrid-Suspension mit Pentan aus und trocknet im Vakuum unter Stickstoff. Zum Kaliumhydrid (etwa 25 mMol) gibt man 50 ml Toluol und die Lösung von 2,98 g, 3,5-Dimethoxyphenyl-acetonitril (16,9 mMol) in 30 ml Toluol, rührt 15 Minuten bei Raumtemperatur und tropft dann die Lösung von 6,4 g 1$\beta$ - tert. - Butoxy - 7a$\beta$ - methyl - 4 - (phenyl - sulfonyl - methyl) - perhydroinden - 5 - on(16,9mMol) in Toluol zu. Nach 2 Stunden Rühren wird die Mischung mit Eisessig angesäuert, in Chloroform aufgenommen und dann mit gesättigter Bicarbonatlösung und mit Wasser ausgewaschen. Nach Trocknen über Magnesiumsulfat und Eindampfen werden 8,72 g des Stereoisomerengemisches erhalten. Für die anschließende Cyclisierung ist dieses Rohprodukt rein genug. Zur Strukturaufklärung wird eine Probe durch präparative Schichtchromatographie (Kieselgel, Laufmittel: Cyclohexan/Essigester 8:2) in die beiden Komponenten aufgetrennt:

17$\beta$ - tert. - Butoxy - 6$\alpha$ - cyan - 1,3 - dimethoxy - 9,10 - seco - 1,3,5 - (10) - östra - trien - 9 - on, ölig $[\alpha]_D = +20,4°$ (0,5% in Chloroform); 17$\beta$ - tert. - Butoxy - 6$\beta$ - cyan - 1,3 - dimethoxy - 9,10 - seco - 1,3,5(10) - östratrien - 9 - on, ölig, $[\alpha]_D = +5,2°$ (0,5% in Chloroform.

b) 17$\beta$-tert.-Butoxy-6-cyan-1,3-dimethoxy-1,3,5(10),9(11)-östratetraen

Die Lösung von 16,2 g 17$\beta$ - tert. - Butoxy - 6 - cyan - 1,3 - dimethoxy - 9,10 - seco - 1,3,5(10) - östratrien - 9 - on (Rohprodukt und Isomerengemisch in 6) löst man in 200 ml Äthanol und erwärmt mit einer Lösung von 0,4 ml konzentrierter Schwefelsäure in 40 ml Äthanol 20 Stunden auf 60°. Das Lösungsmittel wird etwa zur Hälfte abdestilliert, der Rückstand in Chloroform aufgenommen un mit Wasser ausgeschüttelt. Nach Trocknen über Magnesiumsulfat und Eindampfen bleiben 13,8 g des Titelgemisches. Durch Kristallisation aus Essigester/Diisopropyläther lassen sich 4,81 g der 6$\alpha$-Cyan-Verbindung (37%) erhalten. Schmelzpunkt: 172—176°C, $[\alpha]_D = +106,1°$ (0,5% in Chloroform). Durch Chromatographie an Kieselgel (Gradient Benzin/Essigester 95:5 → 60:40) und durch anschließende präparative Schichtchromatographie läßt sich die 6$\beta$-Cyanverbindung darstellen.

c) 17$\beta$-tert.-Butoxy-6-cyan-1,3-dimethoxy-1,3,5(10)-östratrien

Mal löst 17,7 g 6$\alpha$-Nitril der Verfahrensstufe b) in 400 ml Äthanol und 200 ml Benzol, fügt 160 ml Lithiumhydrid und 7,5 g Palladium/Kohle (10%) zu und hydriert bis 1 Liter Wasserstoff aufgenommen sind. Nach Filtration und Abziehen des Lösungsmittels bleiben 17,4 g Rohprodukt. Dieses Stereoisomerengemisch kann bei 0,1 Torr und 245—250°C am Kugelrohr destilliert werden. Ausbeute 15,8 g (89%). Eine Probe der Mischung wird durch präparative Schichtchromatographie (Kieselgel) durch zweimaliges Entwicklen mit Benzin/Äther 8:2 getrennt. Selbst die reinen Verbindungen bilden keine brauchbaren Kristalle.

6$\alpha$-Nitril $[\alpha]_D = +129°$ (0,5% in Chloroform);
6$\beta$-Nitril $[\alpha]_D = +124°$ (0,5% in Chloroform).

d) 17$\beta$-tert.-Butoxy-1,3-dimethoxy-1,3,5(10)-östratrien-6-on

13,2 g des gut getrockneten, möglichst destillierten Gemisches der Nitrile nach Beispiel 1 c) (33 mMol) löst man in 200 ml absolutem DMF und gibt 2,4 g 80%iges Natriumhydrid (100 mMol) zu. Man erwärmt unter Rühren zwei Stunden auf 50° und leitet dann bei Raumtemperatur etwa 10 Minuten lang Sauerstoff durch die Lösung. Die Reaktionsmischung wird nach der Luftoxydation vorsichtig mit Eisessig angesäuert, in Wasser aufgenommen und fünfmal mit Äther extrahiert. Nach Trocknen und Eindampfen, zuletzt bei 50°C im Hochvakuum, erhält man 17,2 g Rohprodukt. Aus wenig Benzin kristallisieren bei Raumtemperatur 6,16 g (44%), Schmelzpunkt 104—108°C, $[\alpha]_D = +97°$ (0,5% in Chloroform).

Die Mutterlauge wird an Kieselgel mit Benzin/Essigester (Gradient 95:5 → 1:1) chromatographiert und die betreffenden Fraktionen aus Benzin/Diisopropyläther kristallisiert, Ausbeute 2,9 g (20%), Schmelzpunkt 105—107°C $[\alpha]_D = +97°$ (0,5% in Chloroform).

Beispiel 2

17$\beta$-tert.-Butoxy-6$\alpha$-cyan-1,3-dimethoxy-1,3,5(10),9(11)-östratetraen

In 200 ml Toluol löst man 17,7 g 3,5-Dimethoxyphenyl-acetonitril (100 mMol) und 37,8 g 1$\beta$ - tert.-Butoxy - 7a$\beta$ - methyl - 4 - (phenylsulfonyl - methyl) - perhydroinden - 5 - on (100 mMol), gibt 5,0 g Benzyl-triäthyl-ammoniumchlorid zu und tropft unter Rühren langsam 40 ml einer 50%igen Kalilauge zu. Man rührt bei Raumtemperatur über Nacht, verdünnt dann mit Äther, wäscht die organische Phase mit Wasser neutral und erhält nach Trocknen und Eindampfen 48,3 g Secoverbindung des Beispiels 1. Ohne weitere Reinigung löst man diese Mischung in 750 ml Methanol und 50 ml 10 n

Salzsäure und rührt über Nacht bei Raumtemperatur. Der Niederschlag wird abgesaugt und getrocknet, Ausbeute 19,7 g (49,6 %). Schmelzpunkt 165—168°C $[\alpha]_D = +98°$ (0,5% in Chloroform). Die Mutterlauge wird weitgehend eingedampft, der Rückstand in Chloroform aufgenommen und zweimal mit Wasser gewaschen. Nach Trocknen mit Magnesiumsulfat und Eindampfen löst man in 300 ml Methanol, fügt 0,7 g Natriummethylat zu und rührt 5 Stunden bei Raumtemperatur. Der Niederschlag wird abfiltriert und getrocknet, Ausbeute 7,05 g (17,7%), Schmelzpunkt 167—170°C $[\alpha]_D = +99°$ (0,5% in Chloroform). Die reine Verbindung wird durch Umkristallisation aus Essigester/Diisopropyläther erhalten. Schmelzpunkt 172—176°C, $[\alpha]_D = +106°$ (0,5% in Chloroform).

Die Weiterverarbeitung erfolgt analog Beispiel 1.

Beispiel 3

17β-tert.-Butoxy-1,3-dimethoxy-1,3,5(10)-östratrien-6-on

In 120 ml Toluol löst man 9,4 g rohes, undestilliertes Gemisch der Verbindungen nach Beispiel 1 c), gibt 5,6 g Benzyl-triäthyl-ammonium-chlorid (25 mMol) und 50 ml 50%ige Kalilauge zu. Man rührt die zwei Phasen kräftig und leitet Sauerstoff durch die Mischung. Nach 6 Stunden ist die Reaktion beendet, man verdünnt mit Äther, wäscht mit Wasser neutral, trocknet mit Magnesiumsulfat und dampft ein. Aus dem Rohprodukt von 7,1 g kristallisieren aus Benzin/Diisopropyläther 3,4 g Keton aus (46%), Schmelzpunkt 101—102°C. Die Mutterlauge wird an Kieselgel mit einem Gradienten von Benzin/Essigester (95:5 70:30) chromatographiert und man erhält nach Kristallisation weitere 1,6 g Keton (22%) der gleichen Qualität.

Umkristallisation aus Diisopropyläther und Benzin liefert ein reines Produkt, Schmelzpunkt 105—107°C, $[\alpha]_D = +97°$ (0,5% in Chloroform).

Beispiel 4

17β-tert.-Butoxy-1,3-dimethoxy-7α-methyl-1,3,5-östratrien-6-on

In 300 ml Dimethoxyäthan löst man 61,5 g rohes Keton nach Beispiel 1 d) (etwa 150 mMol), die man zu einer Suspension von 5,9 g Natriumhydrid (80%, ölfrei gewaschen mit Pentan) (200 mMol) in 50 ml Dimethoxyäthan gibt. Man fügt 12,7 ml Methyljodid (200 mMol) zu und erwärmt zwei Stunden auf 60°C. Die Mischung wird nach Abkühlen vorsichtig mit Wasser versetzt, ein Teil des Lösungsmittels abdestilliert, der Rückstand mit Äther aufgenommen und mit Wasser gewaschen. Die Ätherphase trocknet man mit Magnesiumsulfat, dampft ein und erhält 62,4 g Rohprodukt, das nicht gereinigt, sondern gleich der Wolff-Kishner Reduktion unterworfen wird. Durch Umkristallisation aus Essigester/-Diisopropyläther erhält man reines 17β - tert. - Butoxy - 1,3 - dimethoxy - 7α - methyl - 1,3,5(10) - östratrien - 6 - on, Schmelzpunkt 139—141°C $[\alpha]_D = +65°$ (0,5% in Chloroform).

Beispiel 5

17β-tert.-Butoxy-1,3-dimethoxy-7α-methyl-1,3,5(10)-östratrien

4,0 g Keton nach Beispiel 4 (10 mMol) löst man in einer Mischung aus 40 ml Hydrazinhydrat (80%ig), 8,4 g Hydrazinhydrochlorid und 200 ml Triäthylenglykol und erhitzt 2,5 Stunden auf 130°C. Man gibt nun nach und nach 12,3 g pulverisiertes Kaliumhydroxid zu, wobei die Temperatur langsam auf 200°C gesteigert wird. Nach 1—2 Stunden ist die Reaktion beendet, man läßt abkühlen, nimmt in Wasser auf und extrahiert mit Äther. Die organisch Phase wird mit Wasser neutralgewaschen, über Magnesiumsulfat getrocknet und eingedampft. Das Rohprodukt (5,15 g) wird aus Diisopropyläther und Benzin kristallisiert. Man erhält zwei Kristallfraktionen 2,7 g (70%), Schmelzpunkt 135—136°C, $[\alpha]_D = +126°$ (0,5% in Chloroform);
0,27 g (12%), Schmelzpunkt 133—134°C, $[\alpha]_D = +125°$ (0,5% in Chloroform).

Beispiel 6

a) 1,3-Dimethoxy-7α-methyl-1,3,5(10)-östratrien-17β-ol-acetat

9,75 g 17β-tert.-Butoxy-1,3-dimethoxy-7α-methyl-1,3 (10)-östratrien (25 mMol) löst man in 200 ml Eisessig, gibt 2 ml konzentrierte Schwefelsäure zu und läßt über Nacht bei Raumtemperatur stehen. Dann wird im Vakuum die Hauptmenge der Essigsäure abdestilliert, in gesättigter Natriumchloridlösung aufgenommen und mit Essigester ausgeschüttelt. Die organische Phase wird mit Magnesiumsulfat getrocknet und eingedampft. Der Rückstand (10,15 g) wird in der Wärme aus Diisopropyläther und Benzin kristallisiert. Man erhält 7,6 g (81%), Schmelzpunkt 129—131°C, $[\alpha]_D = +112°$ (0,5% in Chloroform).

b) 1,3-Dimethoxy-7α-methyl-1,3,5(10)-östratrien-17β-ol

In 200 ml Äthanolund 15 ml 4 n Natronlauge löst man 7,6 g des nach Beispiels 6 a) erhaltenen Acetats (20 mMol) und erwärmt eine halbe Stunde auf 60°C. Dann wird das Lösungsmittel weitgehend abdestilliert und in Natriumchloridlösung und Essigester aufgenommen. Die mit Wasser neutralgewaschene und mit Magnesiumsulfat getrocknete Lösung hinterläßt nach dem Eindampfen 6,91 g Rohprodukt, das aus Diisopropyläther 4,15 g kristalline Titelverbindung (62%) Schmelzpunkt 82—83°C, $[\alpha]_D = +128°$ (0,5% in Chloroform) und 1,40 g kristalline Titelverbindung (21%), Schmelzpunkt 79—80°C, $[\alpha]_D = +131°$ (0,5% in Chloroform) liefert.

c) 1,3-Dimethoxy-7$\alpha$-methyl-1,3,5(10)-östratrien-17-on

In 1 l wasserfreiem Toluol und 200 ml Cyclohexanon löst man 17,35 g 1,3 - Dimethoxy - 7$\alpha$ - methyl - 1,3,5(10) - östratrien - 17$\beta$ - ol (nach Beispiel 6 b)) (52,5 mMol) und destilliert 100 ml ab. Dann gibt man die Lösung von 7,9 g Aluminium-isopropylat in 250 ml absolutem Toluol langsam zu, wobei weiter Toluol aus der Mischung abdestilliert wird. Nach etwa 2 Stunden läßt man abkühlen, schüttelt dreimal mit 2 n Schwefelsäure und dann mit Wasser aus, trocknet die organische Phase mit Magnesiumsulfat und dampft ein. Aus dem öligen Rückstand scheiden sich Kristalle ab, die aus Diisopropyäther umkristallisiert werden. Vom Filtrat wird im Hochvakuum bei 160 bis 170°C am Kugelrohr alles Flüchtige abdestilliert und der kristalline Rückstand (23,9 g) aus Diisopropyläther umkristallisiert. Man erhält in insgesamt 12,9 g (75%), Schmelzpunkt 212—214°C, $[\alpha]_D = +225°$ (0,5% in Chloroform).

d) 1,3-Diacetoxy-7$\alpha$-methyl-1,3,5(10)-östratrien-17-on

In 50 ml einer konzentrierten Lösung von Bromwasserstoff in Eisessig und 10 ml Wasser löst man 1,0 g 1,3-Dimethoxy-7$\alpha$-methyl-1,3,5(10)-östratrien-17-on, 5 g Lithiumjodid und 1,0 g Zinn-II-chlorid und erwärmt unter Argon 3,5 Stunden auf 60°C. Man läßt die Reaktionsmischung abkühlen, fügt ein weiteres Gramm Zinn-II-chlorid zu und versetzt bei —20°C tropfenweise mit 100 ml Essigsäureanhydrid. Man gibt bei 0°C 2 g Natriumacetat zu und erwärmt 1,5 Stunden lang auf 80°C. Die Mischung wird auf Eis gegossen, etwa eine Stunde lang gerührt und dann mit Essigester viermal ausgeschüttelt. Die organische Phase wird getrocknet und eingedampft. Aus dem Rohprodukt von 1,2 g werden durch Kristallisation aus Diisopropyläther und Benzin 0,92 g isoliert (79%), Schmelzpunkt 162—164°C, $[\alpha]_D = +183°$ (0,5% in Chloroform).

Beispiel 7

a) 17$\beta$-tert.-Butoxy-6-carbomethoxy-1,3-dimethoxy-9,10-seco-1,3,5(10)-östratrien-9-on

2.50 g Kaliumhydrid Suspension (25%ig; etwa 10 mMol) wäscht man ölfrei, gibt unter Argon 100 ml Toluol und die Lösung von 2,1 g (3,5-Dimethoxyphenyl)-essigsäure-methylester (10 mMol) in 20 ml Toluol zu. Nach 15 minütigem Rühren versetzt man mit einer Lösung von 3,78 g 1$\beta$ - tert. - Butoxy - 7a$\beta$ - methyl - 4 - (phenylsulfonyl-methyl) - 3a,4,5,6,7,7a - hexahydroindan - 5 - on (10 mMol) in 50 ml Toluol und rührt weitere 2 Stunden. Anschließend wird mit Essigsäure angesäuert, mit gesättigter Bicarbonatlösung ausgeschüttelt und nach Trocknen eingedampft. Das Rohprodukt ist eine Mischung der beiden Isomeren und wird ohne Reinigung weiterverarbeitet.

b) 17$\beta$-tert.-Butoxy-6-carbomethoxy-1,3-dimethoxy-1,3,5(10),9-(11)-östratetraen

Das oben erhaltene Rohprodukt löst man in 100 ml Aceton, gibt unter Eiskühlung 0,2 ml konzentrierte Schwefelsäure zu, und läßt 20 Stunden bei Raumtemperatur rühren.6 Dann dampft man weitgehend ein, versetzt mit Chloroform und schüttelt mit gesättigter Bicarbonatlösung und mit Wasser aus. Das erhaltene Rohprodukt wird durch Chromatographie an Kieselgel in die beiden Isomeren getrennt. Die 6$\beta$-Carboxymethylverbindung schmilzt bei 152—154°C.

c) 17$\beta$-tert.-Butoxy-6$\beta$-carbomethoxy-1,3-dimethoxy-1,3,5(10)-östratrien

430 mg 17$\beta$ - tert. - Butoxy - 6$\beta$ - carbomethoxy - 1,3 - dimethoxy - 1,3,5 - (10),9(11) - östratetraen (1 mMol) löst man in 10 ml Methanol, gibt 100 mg Palladium/Kohle (10%) zu und hydriert bei Normaldruck. Man filtriert vom Katalysator ab, dampft das Lösungsmittel ab und kristallisiert aus Essigester und Diisopropyläther, Ausbeute 375 mg, Schmelzpunkt 130°C

d) 17$\beta$-tert.Butoxy-1,3-dimethoxy-1,3,5(10)-östratrien-6-on

Das oben erhaltene Hydrierprodukt löst man in 10 ml wasserfreiem Tetrahydrofuran und gibt die Lösung von Lithiumdiisopropylamid in 10 ml Tetrahydrofuran (dargestellt aus Diisopropylamin und 1 mMol Butyllithiumlösung) unter Eiskühlung zu. In diese Lösung leitet man 15 Minuten lang Sauerstoff ein und versetzt dann mit etwas Eisessig und mit einer Lösung von wäßrigem Natriumsulfit. Die Mischung wird teilweise eingedampft, mit Chloroform extrahiert, diese Phase getrocknet und eingedampft. Das Rohprodukt löst man in 5 ml Methanol und 5 ml 4 n Natronlauge und rührt 2 Stunden bei Raumtemperatur. Man säuert mit 4 n Salzsäure an, extrahiert mit Essigester, wäscht die Essigesterphase mit gesättigter Kochsalzlösung aus, trocknet und destilliert das Solvens ab. Der Rückstand wird ohne weitere Reinigung in 10 ml Essigsäure gelöst und bei Raumtemperatur mit einer Lösung von Chromtrioxid in Essigsäure versetzt. Dann verdünnt man mit Wasser, schüttelt mit Chloroform aus, wäscht die Chloroformphase mit Bicarbonatlösung und Wasser und dampft ein. Aus Benzin und Diisopropyläther erhält man 270 mg der gewünschten Verbindung von Schmelzpunkt 104—107°C, $[\alpha]_D = +97°$ (0,5% in Chloroform).

Beispiel 8

a) 17$\beta$-tert.-Butoxy-1,3-dimethoxy-6-methylsulfonyl-9,10-seco-1,3,5(10)-östratrien-9-on

0,9 g Methyl-(3,5-dimethoxybenzyl)-sulfon (2 mMol) und 756 mg 1$\beta$ - tert. - Butoxy - 7a$\beta$ - methyl - 4 - (phenylsulfonylmethyl) - 3a,4,5,6,7,7a - hexahydroindan - 5 - on (2 mMol) in 20 ml

Toluol gibt man zu 0,5 g 25%igem Kaliumhydrid, das mit Pentan ölfrei gewaschen wurde. Man rührt die Mischung 4 Stunden bei Raumtemperatur, säuert dann mit Eisessig vorsichtig an und schüttelt mit gesättigter Bicarbonatlösung aus. Nach Trocknen und Eindampfen erhält man ein öliges Isomerengemisch.

b) 17β-tert.-Butoxy-1,3-dimethoxy-6-methylsulfonyl-1,3,5-(10),9(11)-östratetraen

Das oben erhaltene Rohprodukt löst man in 15 ml Äthanol, fügt 0,5 ml Schwefelsäure zu und rührt bei Eiskühlung 8 Stunden. Man destilliert die Hauptmenge des Lösungsmittels ab, nimmt in Chloroform auf und schüttelt mit gesättigter Bicarbonatlösung aus. Das Solvens wird abdestilliert und der Rückstand chromatographiert, wobei man 510 mg der 6β-Methylsulfonylverbindung erhält.

c) 17β-tert.-Butoxy-1,3-dimethoxy-6β-methylsulfonyl-1,3,5(10)-östratrien

450 mg 17β-tert.-Butoxy-1,3-dimethoxy-6β-methylsulfonyl-1,3,5(10),9(11)-östratetraen löst man in 20 ml Äthanol und 10 ml Toluol und hydriert mit Palladium/Kohle (10%) als Katalysator. Nach 4 Stunden bei Raumtemperatur wird vom Katalysator abfiltriert und das Lösungsmittel abdestilliert. Der Rückstand kristallisiert aus Essigester/Benzin, Schmelzpunkt 189—193°C.

d) 17β-tert.-Butoxy-1,3-dimethoxy-1,3,5(10)-östratrien-6-on

Man löst 450 mg 17β-tert.-Butoxy-1,3-dimethoxy-6β-methyl-sulfonyl-1,3,5(10)-östratrien (1 mMol) in 20 ml Toluol, unterschichtet mit 8 ml 18 n Natronlauge und gibt 0,5 g Tetrabutylammonium-hydrogensulfat zu. Man rührt bei Raumtemperatur kräftig durch und leitet 20 Minuten lang Sauerstoff durch die Mischung. Dann trennt man die Phasen, schüttelt die organische Phase mit Wasser aus und destilliert das Lösungsmittel ab. Der Rückstand wird an Kieselgel chromatographiert und liefert das gewünschte Produkt in 210 mg Ausbeute, Schmelzpunkt 104—106°C, $[\alpha]_D = +96°$ (0,5% in Chloroform).

Beispiel 9

a) 17β-tert.-Butoxy-3-methoxy-6-cyan-9,10-seco-1,3,5(10)-östratrien-9-on

Man kondensiert 2,01 g 3-Methoxy-phenyl-acetonitril (10 mMol) und 3,78 g 1β - tert - Butoxy - 7aβ - methyl - 4 - (phenylsulfonylmethyl) - 3a,4,5,6,7,7a - hexahydroindan - 5 - on (10 mMol) wie oben beschrieben mit 2,5 g 25%igem Kaliumhydrid in Toluol und erhält nach 6 Stunden Reaktionszeit und Aufarbeitung die gesuchte Verbindung als Öl.

b) 17β-tert.-Butoxy-6-cyan-3-methoxy-1,3,5(10),9(11)-östratetraen

Das rohe Isomerengemisch löst man in 50 ml Toluol und versetzt mit 1 ml Trifluoressigsäure und 3 ml Trifluoressigsäureanhydrid bei Raumtemperatur. Dann wird mit gesättigter Bicarbonatlösung versetzt und die Toluolphase abgetrennt, getrocknet und eingedampft. Das 6β-Cyan-Isomere wird chromatographisch abgetrennt und aus Diisopropyläther kristallisiert, Ausbeute 1,8 g, Schmelzpunkt 145—149°C.

c) 17β-tert.-Butoxy-6β-cyan-3-methoxy-1,3,5(10)-östratrien

Man löst 1,8 g 17β-tert.-Butoxy-6-cyan-3-methoxy-1,3,5(10),9(11)-östratetraen in 40 ml Äthanol, fügt 300 mg Palladium/Kohle (10%) zu und hydriert bei Raumtemperatur und Normaldruck. Nach 2 Stunden wird abfiltriert, das Lösungsmittel abdestilliert und der Rückstand aus Essigester und Diisopropyläther kristallisiert, Ausbeute 1,5 g vom Schmelzpunkt 176—180°C.

d) 17β-tert.-Butoxy-3-methoxy-1,3,5(10)-östratrien-6-on

Man löst 1,0 g 17β-tert.-Butoxy-3-methoxy-1,3,5(10)-östratrien und 0,5 g Tetrabutylammonium-hydrogensulfat in 30 ml Toluol und unterschichtet mit 50%iger Kalilauge. Unter Rühren leitet man 4 Stunden Sauerstoff durch die Mischung, trennt die Phasen und wäscht die Oberphase mit Wasser neutral. Dann engt man ein, chromatographiert an Kieselgel, kristallisiert aus Benzin und Diisopropyläther und erhält 0,72 g vom Schmelzpunkt 95—101°C.

e) 17β-tert.-Butoxy-3-methoxy-7α-methyl-1,3,5(10)-östratrien-6-on

356 mg 17β-tert.-Butoxy-3-methoxy-1,3,5(10)-östratrien-6-on (1 mMol) in 5 ml wasserfreiem Toluol gibt man zu einer Lösung von Lithiumhexamethyl-disilylamid in 5 ml Toluol/Hexan (dargestellt aus Hexamethyldisilazan und 1 mMol Butyllithium in Hexan) bei —20°C. Man läßt auf Raumtemperatur erwärmen und rührt eine Stunde. Dann kühlt man wieder auf —20°C ab, gibt die Lösung von 0,1 ml Methyljodid in 1 ml Hexamethylphosphorsäuretriamid zu und laßt zwei Tage bei —20°C stehen. Dann wird in Äther aufgenommen, mit Wasser ausgewaschen und die organische Phase nach Trocknen eingedampft. Der Rückstand wird an Kieselgel chromatographiert, aus Diisopropyläther kristallisiert und ergibt 150 mg der gewünschten Verbindung vom Schmelzpunkt 110—117°C.

Beispiel 10

7α-Methyl-1,3,5(10)-östra-3,17-diol

In 0,5 ml 80%igem Hydrazinhydrat, 250 mg Hydrazinhydrochlorid und 2 ml Triäthylenglycol löst

man 140 mg 17β - tert. - Butoxy - 3 - methoxy - 7α - methyl - 1,3,5(10) - östratrien - 6 - on und erhitzt 2 Stunden auf 130°C. Darauf gibt man portionsweise 1 g pulverisiertes Kaliumhydroxid zu und erhitzt langsam auf 200°C. Nach einer Stunde läßt man abkühlen, nimmt in Wasser auf und schüttelt mit Chloroform aus. Die organische Phase wird mit Wasser gewaschen, getrocknet und eingedampft, wobei 145 mg Rohprodukt hinterbleiben. Dieses wird ohne weitere Reinigung in 2 ml mit Bromwasserstoff gesättigtem Eisessig gelöst, mit 0,4 ml Wasser, 0,2 g Lithiumjodid und 50 mg Zinn-II-chlorid versetzt und zwei Stunden auf 50°C erhitzt. Man verdünnt mit Wasser, schüttelt mit Chloroform aus und reinigt die organische Lösung mit Wasser. Dann wird getrocknet und eingedampft und der Rückstand mit Essigsäureanhydrid und Natriumacetat aus Äther kristallisiert, Ausbeute 65 mg, Schmelzpunkt 119—120°C.

**Patentansprüche für die Vertragsstaaten: BE, CH, DE, FR, GB, IT, LU, NL, SE**

1. 7α-Methyl-Östrogene der allgemeinen Formel I

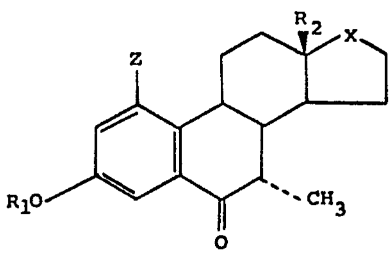

(I)

worin

$R_1$ und $R_2$ eine niedere Alkylgruppe mit 1—4 C-Atomen, Z ein Wasserstoffatom oder eine $OR_1$-Gruppe und X eine funktionell geschützte Hydroxymethylengruppe bedeutet.

2. Verfahren zur Herstellung von 7α-Methyl-Östrogenen der allgemeinen Formel I, gemass Anspruch 1 dadurch gekennzeichnet, daß man in an sich bekannter Weise a) eine Verbindung der allgemeinen Formel II

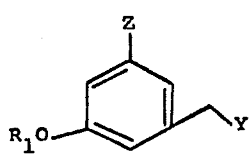

(II)

worin

$R_1$ und Z die in Anspruch 1 angegebene Bedeutung haben und Y ein —C≡N-Gruppe oder eine gegebenenfalls veresterte —COOH— oder —$SO_2R_3$-Gruppe, mit $R_3$ in der Bedeutung einer niederen oder mittleren Alkyl-gruppe mit 1 bis 8 C-Atomen oder einer gegebenenfalls substituierten Arylgruppe darstellt, in Gegenwart eines Deprotonierungsmittels mit einer Verbindung der allgemeinen Formel III

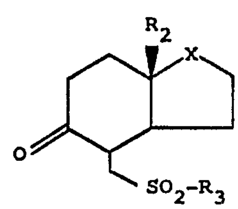

(III)

worin

X, $R_2$ und $R_3$ die in Anspruch 1 angegebene bzw obige Bedeutung haben, umsetzt, wobei eine Verbindung der allgemeinen Formel IV

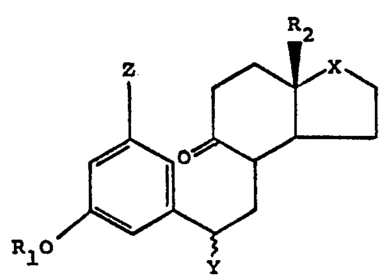

(IV)

worin

$R_1$, $R_2$, X, Z und Y die obige Bedeutung haben, erhalten wird;

b) die Verbindung der allgemeinen Formel IV zu einer Verbindung der allgemeinen Formel V

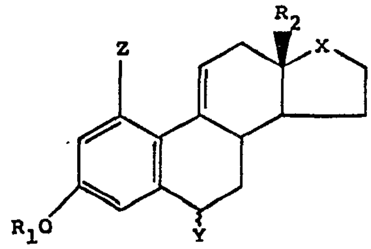

(V)

worin

$R_1$, $R_2$, X, Y und Z die obige Bedeutung haben, cyclisiert;

c) die Verbindung der allgemeinen Formel V zu einer Verbindung der allgemeinen Formel VI

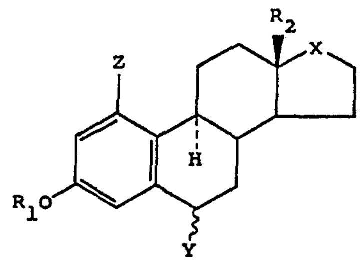

(VI)

worin

$R_1$, $R_2$, X, Y und Z die obige Bedeutung haben, in Gegenwart eines Edelmetallkatalysators hydriert;

d) die Verbindung der allgemeinen Formel VI zu einer Verbindung der allgemeinen Formel VII

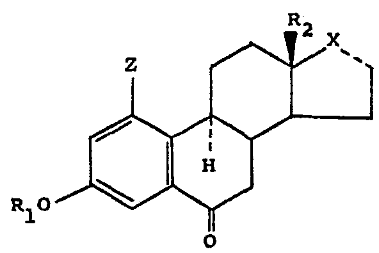

(VII)

worin

$R_1$, $R_2$, Z und X die obige Bedeutung haben oxidiert;

e) die Verbindungen der allgemeinen Formel VII methyliert.

3. Verwendung der Verbindungen der allgemeinen Formel I zur Herstellung der Verbindungen der allgemeinen Formel IX

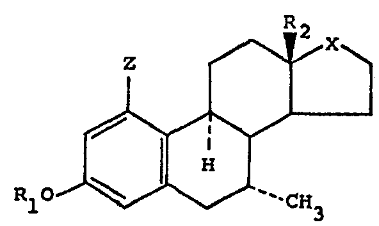

(IX)

worin

$R_1$, $R_2$, Z und X die in Anspruch 1 angegebene Bedeutung haben, indem man die in den Verbindungen der Formel I vorhandene 6-Ketogruppe in an sich bekannter Weise zur Methylengruppe reduziert.

**Revendications pour les Etats contractants: BE, CH, DE, FR, GB, IT, LU, NL, SE**

1. Méthyl-7$\alpha$ oestrogènes répondant à la formule générale I

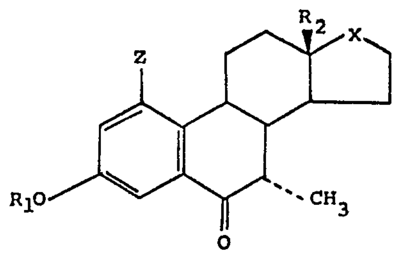

(I)

dans laquelle

$R_1$ et $R_2$ représentent chacun un radical alkyle inférieur contenant de 1 à 4 atomes de carbone, Z représente un atome d'hydrogène ou un radical —$OR_1$ et X représente un radical hydroxyméthylène à groupe hydroxy protégé.

2. Procédé de préparation de méthyl-7$\alpha$ oestrogènes de formule générale (I) selon la revendication 1, procédé caractérisé en ce que, en opérant de manière connue:

a) on fait réagir un composé répondant à la formule générale II:

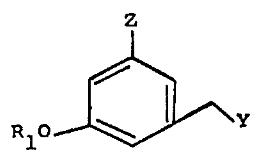

(II)

dans laquelle

$R_1$ et Z ont les significations données à la revendication 1 et Y représente un groupe —C≡N, un groupe —COOH éventuellement estérifié ou un groupe —$SO_2R_3$ dont le symbole $R_3$ désigne un radical alkyle inférieur ou moyen contenant de 1 à 8 atomes de carbone ou un radical aryle éventuellement substitué, en présence d'un agent déprotonant, avec un composé répondant à la formule générale III:

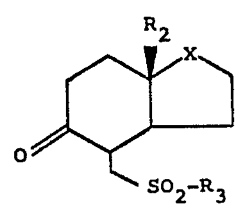

(III)

dans laquelle

X et $R_2$ ont les significations données à la revendication 1 et $R_3$ a la signification donnée ci-dessus, réaction qui conduit à un composé répondant à la formule générale IV:

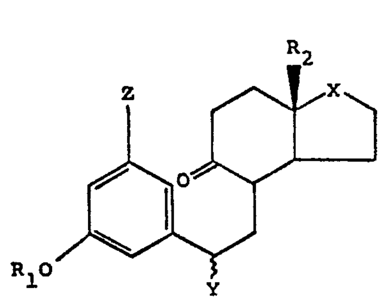

(IV)

dans laquelle

$R_1$, $R_2$, X, Z et Y ont les significations indiquées ci-dessus;

b) on cyclise le composé de formule générale (IV) de manière à la convertir en un composé répondant à la formule générale V:

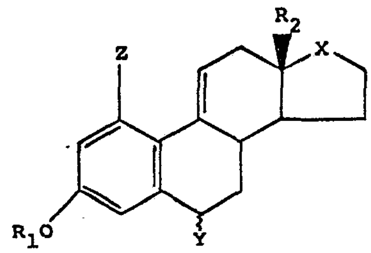

(V)

dans laquelle

$R_1$, $R_2$, X, Y et Z ont les significations précédemment données;

c) on hydrogène le composé de formule générale (V) de manière à le transformer en un composé répondant à la formule générale VI:

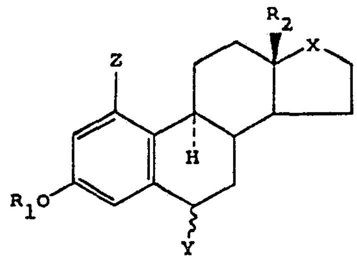

(VI)

dans laquelle

$R_1$, $R_2$, X, Y et Z ont les significations précédemment données, en présence d'un catalyseur à base d'un métal noble;

d) on oxyde le composé de formule générale (VI) pour le transformer en un composé répondant à la formule générale VII:

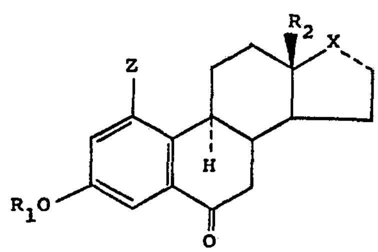

(VII)

dans laquelle

$R_1$, $R_2$, Z et X ont les significations précédemment données; et

e) on méthyle le composé de formule générale (VII).

3. Application des composés de formule générale (I) à la préparation des composés répondant à la formule générale IX:

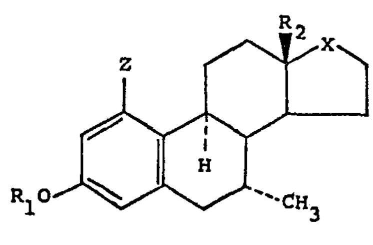

(IX)

dans laquelle

$R_1$, $R_2$, Z et X ont les significations données à la revendication 1, application selon laquelle on réduit en groupe méthylène, de manière connue, le groupe carbonyle en 6 présent dans les composés de formule (I).

**Claims for the Contracting States: BE, CH, DE, FR, GB, IT, LU, NL, SE.**

1. 7$\alpha$-methyl oestrogens of the general formula I

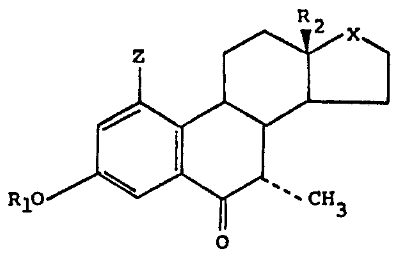

(I)

in which

$R_1$ and $R_2$ represent a lower alkyl group having from 1 to 4 carbon atoms, Z represents a hydrogen atom or an $OR_1$ group, and

X represents a functionally protected hydroxymethylene group.

2. Process for the manufacture of 7$\alpha$-methyl oestrogens of the general formula I according to claim 1, characterised in that, in a manner known *per se*,

a) a compound of the general formula II

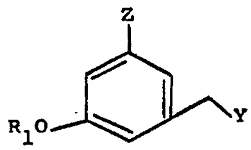

(II)

in which

$R_1$ and Z have the meanings given in claim 1 and Y represents a —C≡N group or an optionally esterified —COOH or —$SO_2R_3$ group, $R_3$ representing a lower or middle alkyl group having from 1 to 8 carbon atoms or an optionally substituted aryl group, is reacted, in the presence of a deprotonating agent, with a compound of the general formula III

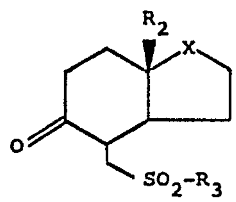

(III)

in which

X and $R_2$ have the meanings given in claim 1 and $R_3$ has the meaning given above, yielding a compound of the general formula IV

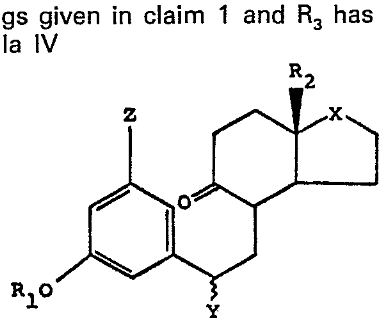

(IV)

in which

$R_1$, $R_2$, X, Z and Y have the meanings given above;

b) the compound of the general formula IV is cyclised to form a compound of the general formula V

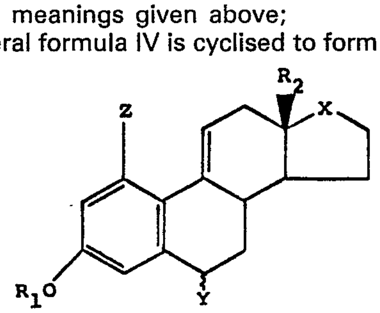

(V)

in which

$R_1$, $R_2$, X, Y and Z have the meanings given above;

c) the compound of the general formula V is hydrogenated in the presence of a noble metal catalyst to form a compound of the general formula VI

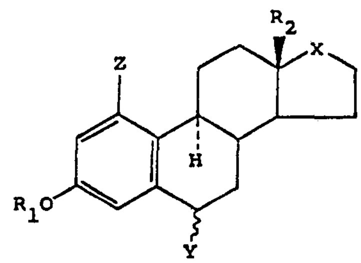

(VI)

in which

$R_1$, $R_2$, X, Y and Z have the meanings given above;

d) the compound of the general formula VI is oxidised to form a compound of the general formula VII

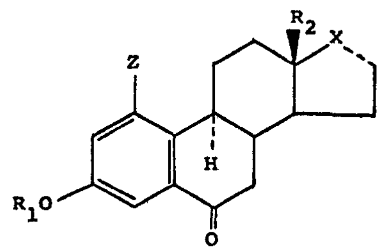

(VII)

in which

$R_1$, $R_2$, Z and X have the meanings given above;

e) the compounds of the general formula VII are methylated.

3. Use of compounds of the general formula I for the manufacture of compounds of the general formula IX

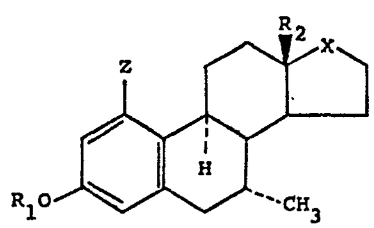

(IX)

in which

$R_1$, $R_2$, Z and X have the meanings given in claim 1, by reacting the 6-keto group present in the compounds of the formula I to form a methylene group in a manner known *per se*.

**Patentanspruch für den Vertragsstaat: AT.**

1. Verfahren zur Herstellung von 7α-Methyl-Östrogenen der allgemeinen Formel I

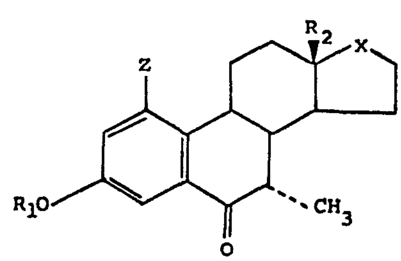

(I)

worin

$R_1$ und $R_2$ eine niedere Alkylgruppe mit 1 bis 4 C-Atomen, Z ein Wasserstoffatom oder eine $OR_1$-Gruppe und X eine funktionell geschützte Hydroxymethylengruppe bedeutet, dadurch gekennzeichnet, daß man

a) eine Verbindung der allgemeinen Formel II

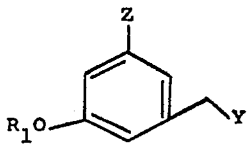

(II)

worin

$R_1$ und Z die obige Bedeutung haben und Y ein —C≡N-Gruppe oder eine gegebenenfalls veresterte —COOH— oder —$SO_2R_3$-Gruppe, mit $R_3$ in der Bedeutung einer niederen oder mittleren Alkyl-gruppe mit 1 bis 8 C-Atomen oder einer gegebenenfalls substituierten Arylgruppe darstellt, in Gegenwart eines Deprotonierungsmittels mit einer Verbindung der allgemeinen Formel III

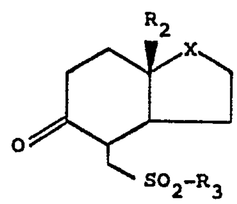

(III)

worin

X, $R_2$ und $R_3$ die obige Bedeutung haben, umsetzt, wobei eine Verbindung der allgemeinen Formel IV

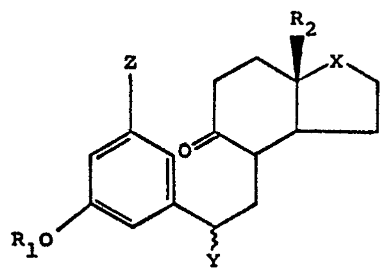

(IV)

worin

$R_1$, $R_2$, X, Z und Y die obige Bedeutung haben, erhalten wird;

b) die Verbindung der allgemeinen Formel IV zu einer Verbindung der allgemeinen Formel V

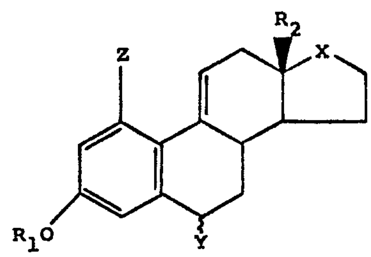

(V)

worin

$R_1$, $R_2$, X, Y und Z die obige Bedeutung haben, cyclisiert;

c) die Verbindung der allgemeinen Formel V zu einer Verbindung der allgemeinen Formel VI

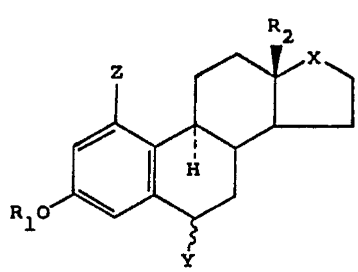

(VI)

worin

$R_1$, $R_2$, X, Y und Z die obige Bedeutung haben, in Gegenwart eines Edelmetallkatalysators hydriert;

d) die Verbindung der allgemeinen Formel VI zu einer Verbindung der allgemeinen Formel VII

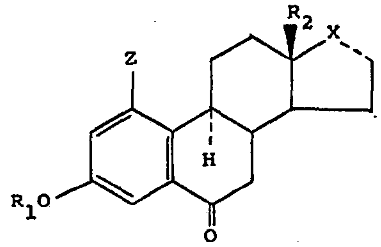

(VII)

worin

$R_1$, $R_2$, Z und X die obige Bedeutung haben oxidiert;

e) die Verbindungen der allgemeinen Formel VII methyliert.

2. Verwendung der gemäß Anspruch 1 erhaltene Verbindungen der allgemeinen Formel I zur Herstellung der Verbindungen der allgemeinen Formel IX

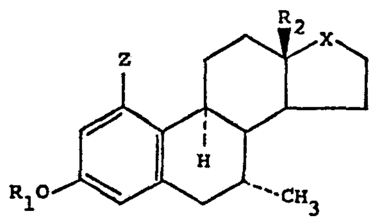

(IX)

worin

$R_1$, $R_2$, Z und X die in Anspruch 1 angegebene Bedeutung haben, indem man die in den Verbindungen der Formel I vorhandene 6-Ketogruppe in an sich bekannter Weise zur Methylengruppe reduziert.

**Revendications pour l'Etat contractant: AT.**

1. Procédé de préparation de méthyl-7$\alpha$ oestrogènes de formule générale (I)

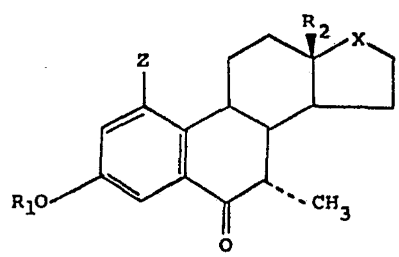

(I)

dans laquelle

$R_1$ et $R_2$ représentent chacun un radical alkyle inférieur contenant de 1 à 4 atomes de carbone, Z représente un atome d'hydrogène ou un radical —$OR_1$ et X représente un radical hydroxyméthylène à groupe hydroxy protégé, procédé caractérisé en ce que, en opérant de manière connue:

a) on fait réagir un composé répondant à la formule générale II:

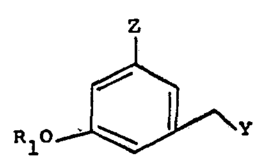

(II)

dans laquelle

$R_1$ et Z ont les significations données à la revendication 1 et Y représente un groupe —C≡N, un groupe —COOH éventuellement estérifié ou un groupe —$SO_2R_3$ dont le symbole $R_3$ désigne un radical alkyle inférieur ou moyen contenant de 1 à 8 atomes de carbone ou un radical aryle éventuellement substitué, en présence d'un agent déprotonant, avec un composé répondant à la formule générale III:

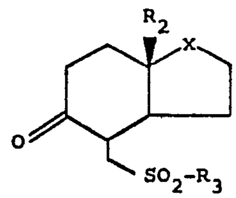

(III)

dans laquelle

X et $R_2$ ont les significations données à la revendication 1 et $R_3$ a la signification donnée ci-dessus, réaction qui conuit à un composé répondant à la formule générale IV:

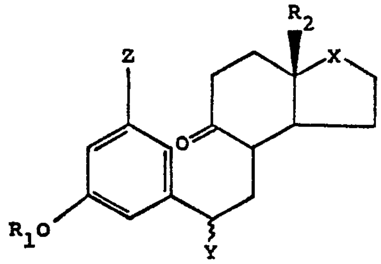

(IV)

dans laquelle

$R_1$, $R_2$, X, Z et Y ont les significations indiquées ci-dessus;

b) on cyclise le composé de formule générale (IV) de manière à la convertir en un composé répondant à la formule générale V:

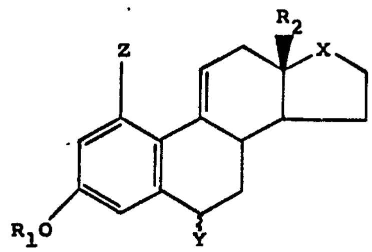

(V)

dans laquelle

$R_1$, $R_2$, X, Y et Z ont les significations précédemment données;

c) on hydrogène le composé de formule générale (V) de manière à le transformer en un composé répondant à la formule générale VI:

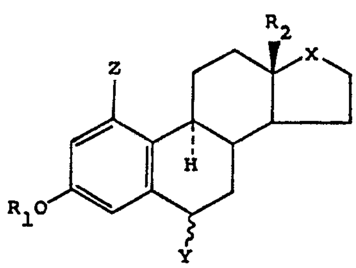

(VI)

dans laquelle

$R_1$, $R_2$, X, Y et Z ont les significations précédemment données, en présence d'un catalyseur à base d'un métal noble;

d) on oxyde le composé de formule générale (VI) pour le transformer en un composé répondant à la formule générale VII:

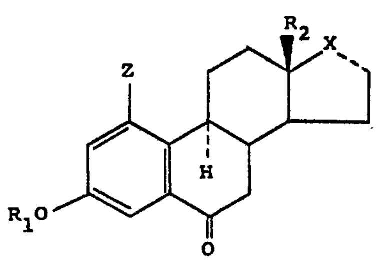

(VII)

dans laquelle

$R_1$, $R_2$, Z et X ont les significations précédemment données; et

e) on méthyle le composé de formule générale (VII).

2. Application des composés de formule générale (I) à la préparation des composés répondant à la formule générale IX:

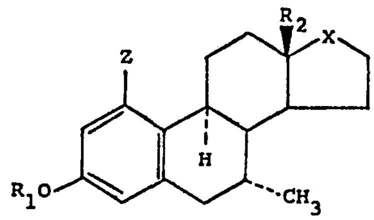

(IX)

dans laquelle

$R_1$, $R_2$, Z et X ont les significations données à la revendication 1, application selon laquelle on réduit en groupe méthylène, de manière connue, le groupe carbonyle en 6 présent dans les composés de formule (I).

**Claims for the Contracting State: AT.**

1. Process for the manufacture of 7$\alpha$-methyl oestrogens of the general formula I

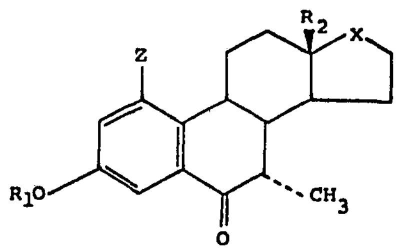

(I)

in which

$R_1$ and $R_2$ represent a lower alkyl group having from 1 to 4 carbon atoms, Z represents a hydrogen atom or an $OR_1$ group, and X represents a functionally protected hydroxymethylene group, characterised in that, in a manner known *per se*,

a) a compound of the general formula II

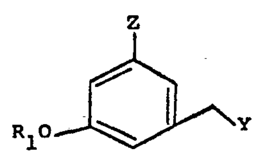

(II)

in which

$R_1$ and Z have the meanings given in claim 1 and Y represents a —C≡N group or an optionally esterified —COOH or —$SO_2R_3$ group, $R_3$ representing a lower or middle alkyl group having from 1 to 8 carbon atoms or an optionally substituted aryl group, is reacted, in the presence of a deprotonating agent, with a compound of the general formula III

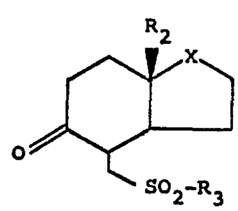

(III)

in which

X and $R_2$ have the meanings given in claim 1 and $R_3$ has the meaning given above, yielding a compound of the general formula IV

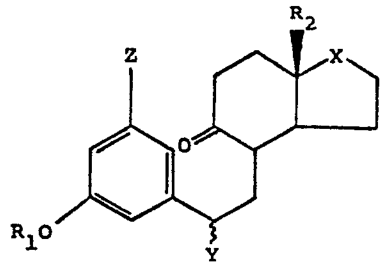

(IV)

in which

$R_1$, $R_2$, X, Z and Y have the meanings given above;

b) the compound of the general formula IV is cyclised to form a compound of the general formula V

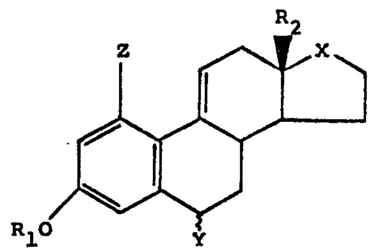

(V)

in which

$R_1$, $R_2$, X, Y and Z have the meanings given above;

c) the compound of the general formula V is hydrogenated in the presence of a noble metal catalyst to form a compound of the general formula VI

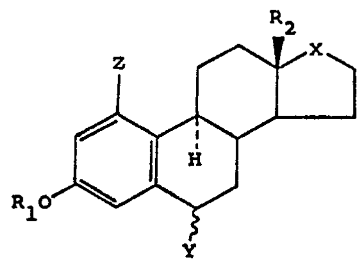

(VI)

in which

$R_1$, $R_2$, X, Y and Z have the meanings given above;

d) the compound of the general formula VI is oxidised to form a compound of the general formula VII

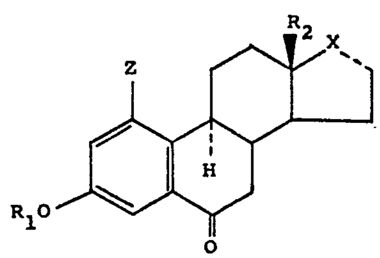

(VII)

in which

$R_1$, $R_2$, Z and X have the meanings given above;

e) the compounds of the general formula VII are methylated.

2. Use of compounds of the general formula I for the manufacture of compounds of the general formula IX

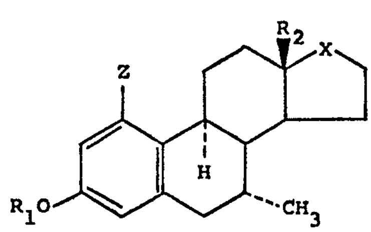

(IX)

in which

$R_1$, $R_2$, Z and X have the meanings given in claim 1, by reacting the 6-keto group present in the compounds of the formula I to form a methylene group in a manner known *per se*.